# EUROPEAN PATENT SPECIFICATION

(11) **EP 0 701 990 B1**
(45) Date of publication and mention of the grant of the patent: **10.03.1999**
(21) Application number: 95306384.9
(22) Date of filing: 12.09.1995
(51) Int. Cl.: C07C 67/37, C07C 69/16, C07C 51/54, C07C 53/12, C07C 69/14, C07C 43/04, C07C 41/09, C07C 47/06, C07C 45/49

(54) **Hydrocarbonylation of dimethyl ether**
Hydrocarbonylierung von Dimethyläther
Hydrocarbonylation de l'éther de diméthyle

(30) Priority: 16.09.1994 US 308018
(43) Date of publication of application: 20.03.1996
(73) Proprietor: AIR PRODUCTS AND CHEMICALS, INC., Allentown, PA 18195-1501 (US)
(72) Inventor: Waller, Francis Joseph, Allentown PA 18103-9670 (US); Studer, David William, Copersburg, PA 18036 (US)
(74) Representative: Burford, Anthony Frederick

(56) References cited:
- EP-A- 0 566 370

## Description

This invention relates to an integrated process for synthesizing ethylidene diacetate, acetic anhydride, acetaldehyde, methyl acetate, and acetic acid, and in particular the production of these oxygenated acetyl compounds from synthesis gas (syngas) via the intermediate compound dimethyl ether.

Ethylidene diacetate (EDDA) is a valuable intermediate in the production of vinyl acetate (VAc), and considerable interest has been focused on developing improved processes for producing ethylidene diacetate. The commercial success of these improved processes, however, requires a market for the acetic acid (HOAc) which is a coproduct of vinyl acetate production. The acetic acid can be sold, esterified with methanol to methyl acetate (MeOAc or MA), or alkylated with dimethyl ether (DME) to methyl acetate and methanol (MeOH). Methyl acetate (MeOAc), acetaldehyde (AcH), and acetic anhydride (Ac₂O) are also important as intermediates for the production of other valuable products.

EP-A-0566370 discloses the production of oxygenated acetyl compounds selected from ethylidene diacetate, acetic acid, acetic anhydride, and methyl acetate from synthesis gas (comprising hydrogen and carbon monoxide) via an intermediate product stream containing dimethyl ether. Dimethyl ether is produced from synthesis gas in a first liquid phase reactor and the reactor effluent comprising dimethyl ether, methanol, and unreacted synthesis gas flows to a second liquid phase reactor containing acetic acid in which the oxygenated acetyl compounds are synthesized catalytically. Vinyl acetate and additional acetic acid optionally are produced by pyrolysis of ethylidene diacetate in a separate reactor system. The synthesis gas is preferably obtained by partial oxidation of a hydrocarbon feedstock such as natural gas. Optionally, a portion of the acetic coproduct is recycled to the partial oxidation reactor for conversion into additional synthesis gas.

In the exemplified process of EP-A-0566370 raw synthesis gas (containing 0.5 to 12 vol% carbon dioxide) is produced by partial oxidation of a hydrocarbon feed, preferably natural gas, and carbon dioxide-rich unreacted synthesis gas (containing 60-70 vol%) recycled from the oxygenated acetyl reactor. The carbon dioxide acts as both a reactant and temperature moderant during the partial oxidation and in certain circumstances can eliminate the need for steam. Optionally at least a portion of the carbon dioxide is removed from the intermediate product stream prior to feeding to the oxygenated acetyl reactor. There is no suggestion that the presence of carbon dioxide in the feed to the oxygenated acetyl reactor could improve the yield or selectivity of the oxygenated acetyl product.

EP-A-0566371 discloses the production of ethylidene diacetate by reacting dimethyl ether, hydrogen, carbon monoxide and acetic acid in the presence of a catalyst system consisting essentially of a Group VIII metal, methyl iodide, lithium iodide and, optionally, lithium acetate. The process is reported to be highly selective toward producing ethylidene diacetate. The hydrogen and carbon monoxide reactants can be provided as synthesis gas but no reference is made to the presence of carbon dioxide in said gas.

Surprisingly, it has now been found that the presence of carbon dioxide in the feed to the oxygenated acetyl reactor can improve the yield or selectivity of the oxygenated acetyl product. In particular, when the catalyst contains lithium acetate, the selectivity to ethylidene diacetate is markedly improved if said feed contains carbon dioxide and methanol in a molar ratio of carbon dioxide to methanol of 5 to 12 and, when the catalyst does not contain lithium acetate, the yield of oxygenated acetyl compounds is markedly improved by the presence of carbon dioxide in an amount providing a carbon dioxide to dimethyl ether molar ratio of 0.3 to 1.3.

In a first broad aspect, the present invention provides a process for synthesizing one or more oxygenated acetyl compounds selected from ethylidene diacetate, acetaldehyde, acetic acid, acetic anhydride, and methyl acetate from a feed containing dimethyl ether, hydrogen, carbon monoxide, carbon dioxide and optionally methanol in a liquid phase reactor containing at least acetic acid in the presence of a catalyst system comprising a Group VIII metal, methyl iodide, lithium iodide, and optionally lithium acetate, characterized in that (a) lithium acetate is not present in the catalyst system and the molar ratio of carbon dioxide to dimethyl ether in said feed is between 0.3 and 1.3 or (b) lithium acetate is present in said catalyst, the molar ratio of carbon dioxide to methanol in said feed is between 5 and 12, and the molar ratio of dimethyl ether to methanol in said feed is between 3 and 11.

In a second broad aspect, the invention also provides the use of carbon dioxide to improve the yield or selectivity of oxygenated acetyl compounds selected from ethylidene diacetate, acetaldehyde, acetic acid, acetic anhydride, and methyl acetate in an oxygenated acetyl process in which a feed containing dimethyl ether, hydrogen, carbon monoxide, and optionally methanol is reacted with acetic acid in a liquid phase reactor in the presence of a catalyst system comprising a Group VIII metal, methyl iodide, lithium iodide, and optionally lithium acetate.

The present invention includes a process for the synthesis of ethylidene diacetate which comprises reacting a feed containing dimethyl ether, methanol, and synthesis gas which contains hydrogen, carbon monoxide, and carbon dioxide in a liquid phase reactor containing at least acetic acid and a catalyst system comprising a Group VIII metal, methyl iodide, lithium iodide, and lithium acetate to produce ethylidene diacetate and one or more additional oxygenated acetyl compounds, wherein the molar ratio of carbon dioxide to methanol in the feed is between 5 and 12 and the molar ratio of dimethyl ether to methanol in the feed is between 3 and 11. The one or more additional oxygenated acetyl compounds can include acetaldehyde, acetic acid, acetic anhydride, and methyl acetate.

The invention also includes the use of carbon dioxide to improve the selectivity to ethylidene diacetate in an oxygenated acetyl process in which a feed containing dimethyl ether, methanol, hydrogen, and carbon monoxide is reacted with acetic acid in a liquid phase reactor in the presence of a catalyst comprising a Group VIII metal, methyl iodide, lithium iodide and lithium acetate.

The invention also provides a process for synthesizing one or more oxygenated acetyl compounds selected from ethylidene diacetate, acetaldehyde, acetic acid, acetic anhydride, and methyl acetate from a feed containing dimethyl ether, hydrogen, carbon monoxide, carbon dioxide and optionally methanol in a liquid phase reactor containing at least acetic acid and a catalyst system comprising a Group VIII metal, methyl iodide, and lithium iodide but not lithium acetate, wherein carbon dioxide is present in said feed such that the molar ratio of carbon dioxide to dimethyl ether is between 0.3 and 1.3.

The invention also provides the use of carbon dioxide to improve the yield of oxygenated acetyl compounds selected from ethylidene diacetate, acetaldehyde, acetic acid, acetic anhydride, and methyl acetate in an oxygenated acetyl process in which a feed containing dimethyl ether, hydrogen, carbon monoxide, and optionally methanol is reacted with acetic acid in a liquid phase reactor in the presence of a catalyst system comprising a Group VIII metal, methyl iodide, and lithium iodide but not lithium acetate.

Usually, it is preferred that the molar ratio of dimethyl ether to methanol in said feed is between 3 and 11 and/or that the molar ratio of carbon monoxide to hydrogen in said feed is between 0.6 and 4.0.

The liquid in said liquid phase reactor usually will comprise one or more of oxygenated acetyl compounds.

The synthesis gas suitably is produced in a partial oxidation reactor system by the partial oxidation of a carbonaceous feedstock, for example methane, natural gas, C₂⁺ gaseous hydrocarbons, naphtha, gas oil, vacuum residuum, petroleum coke, or coal.

It is preferred that the feed to the liquid phase oxygenated acetyl reactor comprises a product stream obtained by reacting synthesis gas comprising hydrogen, carbon monoxide, and carbon dioxide in a liquid phase dimethyl ether reactor in the presence of a methanol synthesis catalyst and a methanol dehydration catalyst suspended in an inert liquid, said product stream comprising dimethyl ether, methanol, and unreacted synthesis gas. Suitably, said methanol dehydration catalyst is selected from alumina, silica-alumina, zeolites, solid acids, solid ion exchange resins, and mixtures thereof. More preferably, said methanol synthesis catalyst comprises copper and said methanol dehydration catalyst comprises alumina, wherein the methanol synthesis catalyst is between 75 and 90% of the methanol synthesis catalyst plus methanol dehydration catalyst on a weight basis. Usually, the liquid phase dimethyl ether reactor is operated at a temperature between 440 and 520°F (225 - 270°C), a pressure between 750 and 2,000 psig (5 - 14 MPa), and a gas hourly space velocity of between 3,000 and 15,000 standard liters/(kg catalyst-h).

Unreacted synthesis gas preferably is recycled from the oxygenated acetyl reactor as feedstock to synthesis gas production.

When ethylidene diacetate product is pyrolysed to vinyl acetate, some or all of the acetic acid in the pyrolysis product can be recycled as feedstock to synthesis gas production.

In the accompanying drawings:
Fig. 1 is a block diagram of an integrated process of the present invention;
Fig. 2 is a general flow diagram of a liquid phase dimethyl ether reactor and separation system of the present invention; and
Fig. 3 is a general flow diagram of a liquid phase oxygenated acetyl (LP OA) reactor and separation system of the present invention.

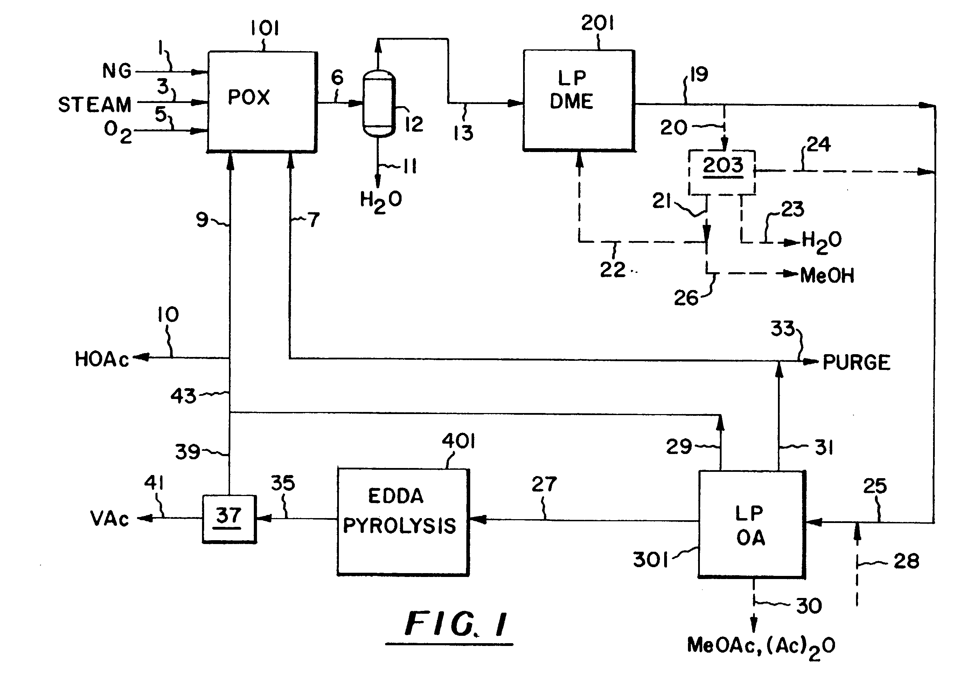

A process block flow diagram for an overall integrated process is shown in Figure 1. This figure differs from Figure 1 of EP-A-0566370 in that the options of (a) direct feed of stream 19 from the liquid phase dimethyl ether (LP DME) reactor system 201 to the LP OA reactor 301 mentioned in EP-A-0566370 and (b) the addition of a carbon dioxide stream 28 are shown. Accordingly, reference should be made to EP-A-0566370 for a description of those parts of present Figure 1 which are common with Figure 1 of EP-A-0566370 and for process details.

The stream 28 allows carbon dioxide 28 to be imported and added to stream 25 to increase the carbon dioxide content. As illustrated in the Examples which follow, the preferred range of the DME/MeOH molar ratio for improved EDDA selectivity is 3 to 11, although higher ratios are expected to give still higher EDDA selectivity. The range of the molar ratio CO₂/MeOH is 5 to 12, preferably 6.0 to 12, more preferably 7 to 12, and especially 8 to 12. The higher ratios in said ranges provide for maximum EDDA selectivity. This preferred mode of operation requires no additional treatment of effluent stream 19 of reactor system 201 as long as the water content is below 2 mol% Water optionally can be removed by condensation if present at higher concentrations. Feed composition, catalyst composition, and operating conditions in DME reactor system 201 can be controlled to yield the preferred composition range in feed 25 to reactor system 301. Optionally CO₂ 28 can be added if required.

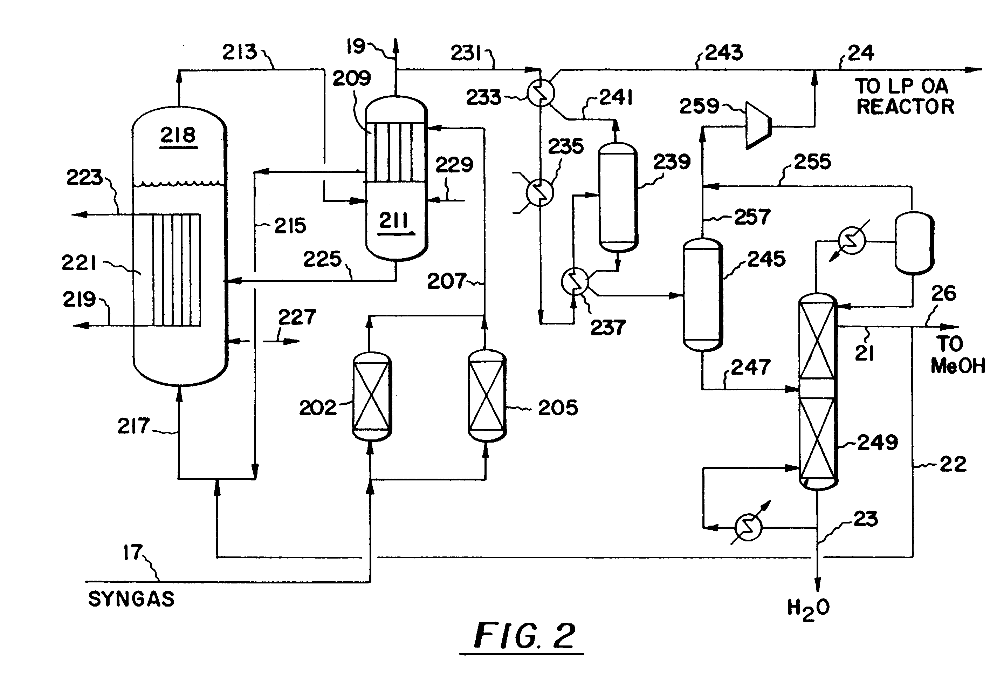

DME reactor system 201 is illustrated in more detail in Fig. 2 and in US-A-5,218,003. This figure differs from Figure 2 of EP-A-0566370 in that it shows the preferred option of feeding LP DME reactor product (19) directly to the LP OA reactor. Accordingly, reference should be made to EP-A-0566370 for a description of those parts of present Figure 2 which are common with Figure 2 of EP-A-0566370

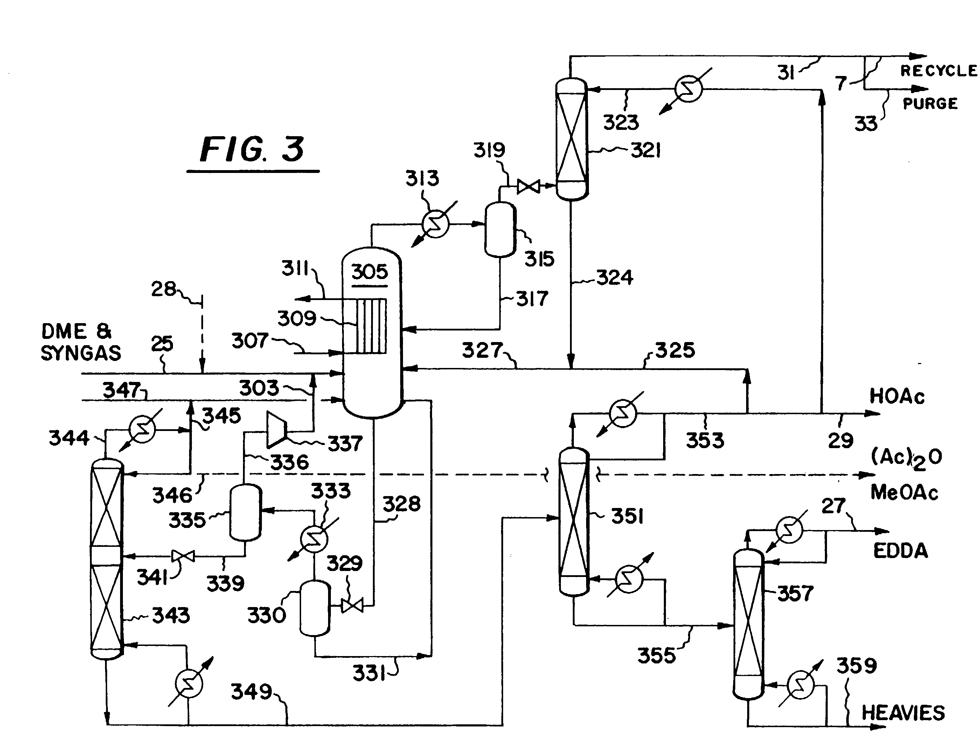

The OA reactor system is illustrated in more detail in Fig. 3. This figure differs from Figure 3 of EP-A-0566370 in that it shows the option of feeding additional carbon dioxide 28 to the LP OA reactor discussed above in connection with Figure 1. Accordingly, reference should be made to EP-A-0566370 for a description of those parts of present Figure 3 which are common with Figure 3 of EP-A-0566370.

The presence of CO₂ in the feed gas can increase acetyl yield, and when methanol is also present in the feed, the presence of CO₂ in the feed increases the selectivity to EDDA. This is illustrated in the Examples which follow.

The following examples are presented to further illustrate the scope of the present invention.

### EXAMPLE 1

Experiments were carried out to investigate the effect of CO₂ on the hydrocarbonylation reactions. The methods and apparatus described in Example 1 of EP-A-0566370 were used; liquid reactants and catalysts were charged to the reactor and the autoclave was flushed with nitrogen once and syngas of the appropriate composition twice. Dimethyl ether was charged and the reactor pressurized to 400 psig (2.75 MPa) with syngas or alternatively to 100 psig (0.6 MPa) with CO₂ followed by syngas up to 400 psig (2.75 MPa). In some experiments, CO₂ was charged by weight from a weighed cylinder. The temperature was increased to the operating temperature of 190°C and the operating pressure was increased and maintained at 1500 psig (10 MPa). After the reaction was complete, the autoclave was depressurized and cooled to ambient conditions. The reaction liquid was isolated and the autoclave was rinsed with 25 cm³ of acetic acid. The reaction liquid and rinse were combined and analyzed by gas chromatography.

Six experiments were carried out using a syngas composition of 80 mol% CO and 20 mol% hydrogen to compare the effect of added CO₂ with and without the catalyst component lithium acetate (LiOAc). The results are given in Table 1. Acetyl yield is defined on a molar basis as the sum of all the observed acetyl products divided by the charged DME. The acetyl products include primarily acetaldehyde (denoted as AcH), methyl acetate (MA), ethyl iodide (EtI), ethyl acetate (EtOAc), acetic anhydride (Ac₂O), and ethylidene diacetate (EDDA). Formation of EDDA requires two moles of DME. It is seen from experiments 1-3 and 4-6 that the addition of CO₂ has a significant effect on the overall acetyl product yield as well as the individual acetyl product selectivities. This finding was unexpected, since none of the prior art teaches that CO₂ influences the product yield and selectivity. US-A-4,810,821 and US-A-5,117,046 actually teach that CO₂ is an inert diluent which does not react with other components present in such a reaction system.

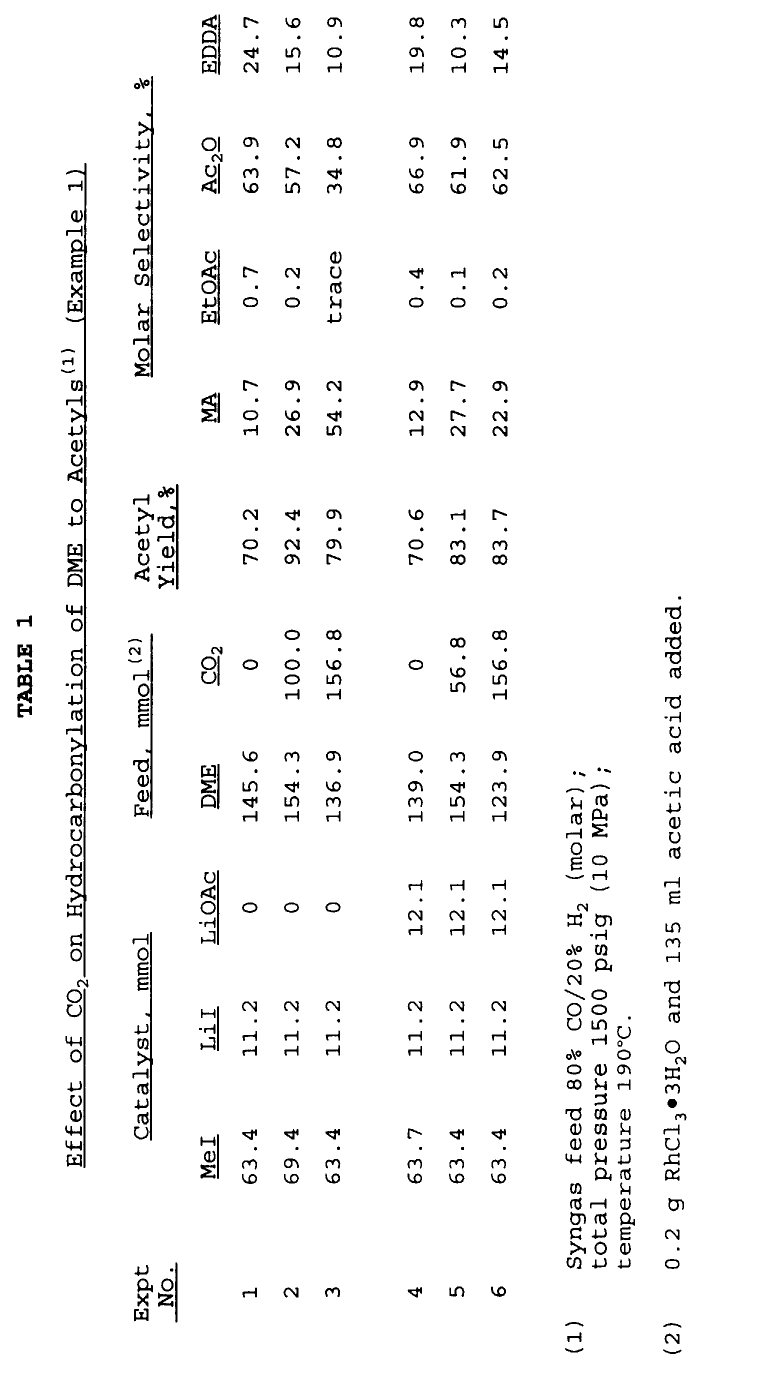

Experiments 1-3, in which lithium acetate was not used as a catalyst component, show that the addition of increasing amounts of CO₂ as a reactant significantly increases the product selectivity to methyl acetate while reducing the selectivity to acetic anhydride and EDDA. Overall yield of acetyl compounds passes through a maximum between zero and 156.8 mmol added CO₂, and is always higher than with reactor operation using syngas containing no CO₂. Experiments 4-6, in which lithium acetate was included as a catalyst component, show similar effects of CO₂ addition although selectivity to acetic anhydride is not decreased significantly.

### EXAMPLE 2

The experiments of Example 1 were repeated except that a syngas containing 50 mol% CO and 50 mol% hydrogen was used. Results are given in Table 2. Without lithium acetate as a catalyst, overall acetyl yield increased significantly as CO₂ was added in experiments 7-9, reaching 96.5% in experiment 9. Decreases in selectivity for acetic anhydride and EDDA were minimal compared with experiments 1-3. When lithium acetate is used as a catalyst component in experiments 10-12, acetyl yield changed only slightly, while EDDA selectivity decreased significantly and methyl acetate selectivity increased significantly.

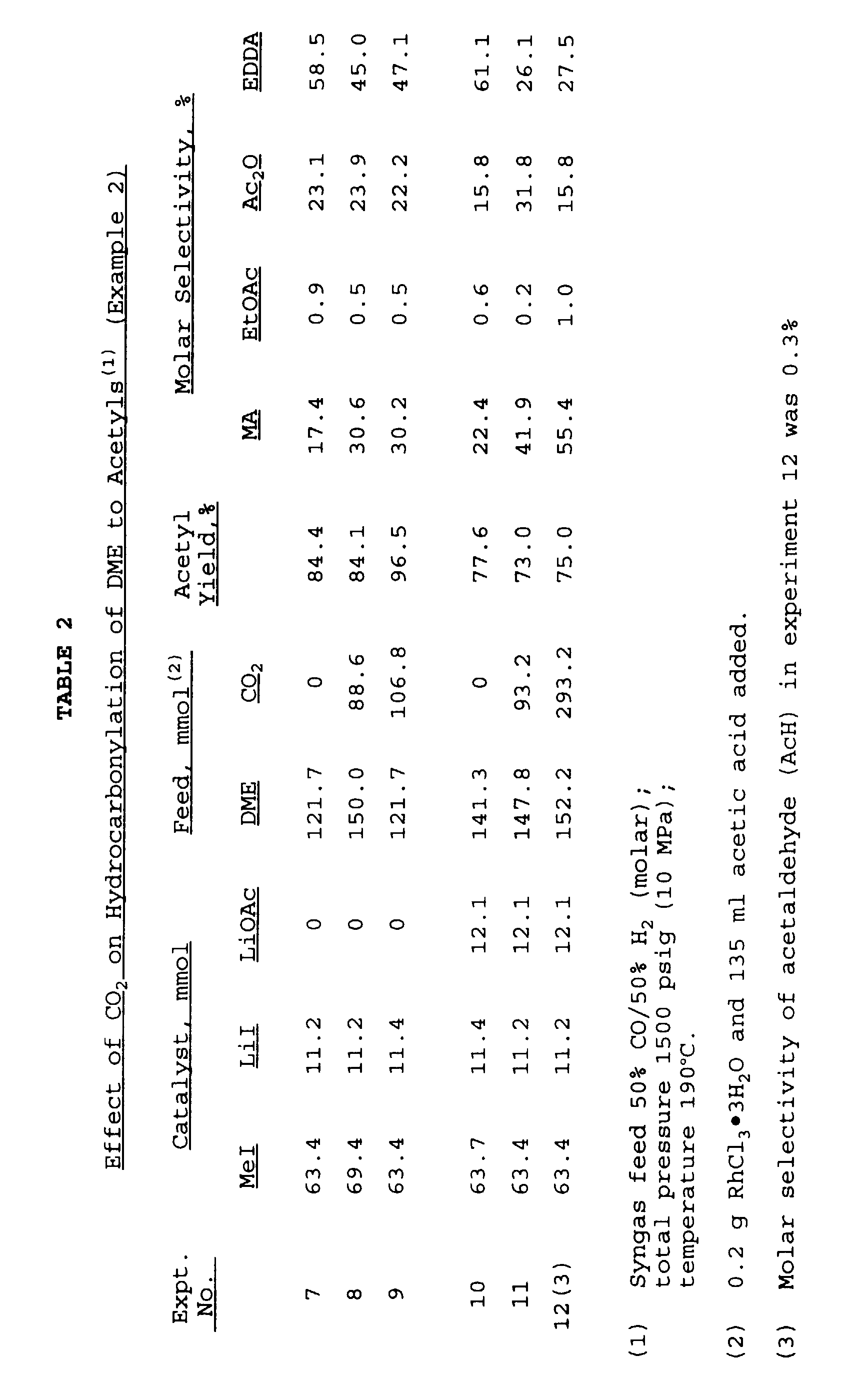

The results of experiments 1-12 are compared in Table 3 in order to observe the effect of the CO₂/DME molar ratio on acetyl yield. The addition of CO₂ to the reactor feed in a preferred CO₂/DME molar ratio range of 0.3-1.3 generally increases the overall acetyl product yield. This indicates unexpectedly that the CO₂ is affecting product yield, because if the CO₂ were merely a diluent the acetyl product yield would decrease due to lower reactant partial pressures. As observed earlier, the addition of CO₂ in this preferred range also unexpectedly changes the selectivities of the various individual acetyl products.

**TABLE 3**

| Effect of CO₂/DME Molar Ratio on Acetyl Yield | | |
|---|---|---|
| Exp. No. | CO₂/DME Molar Ratio | Acetyl Yield, % |
| 1 | 0 | 70.2 |
| 2 | 0.65 | 92.4 |
| 3 | 1.15 | 79.9 |
| | | |
| 4 | 0 | 70.6 |
| 5 | 0.37 | 83.1 |
| 6 | 1.27 | 83.7 |
| | | |
| 7 | 0 | 84.4 |
| 8 | 0.59 | 84.1 |
| 9 | 0.88 | 96.5 |
| | | |
| 10 | 0 | 77.6 |
| 11 | 0.63 | 73.0 |
| 12 | 1.93 | 75.0 |
| | | |

The results of experiments 7-12 confirm the unexpected findings of experiments 1-6, namely, that the addition of CO₂ to a reaction system containing dimethyl ether, CO, and hydrogen has a significant effect on product yield and selectivity. The selection of catalyst components and the amount of CO₂ added can be used to improve overall product yield and change the selectivity of specific product components. When lithium acetate is not present in the catalyst, acetyl yield is increased over that obtained from syngas containing no CO₂ by adding CO₂ such that the molar ratio of CO₂ to DME in the reactor feed is between 0.3 and 1.3.

### EXAMPLE 3

The previous experiments were repeated with methanol added as a reactant to determine the combined effect of CO₂ and methanol on product yield and selectivity. These experiments simulate the operation of liquid phase acetyl reactor in which the feed for LP acetyl reactor system 301 is provided directly from liquid phase dimethyl ether reactor system 201. LP DME reactor effluent 19 contains dimethyl ether, methanol, hydrogen, CO, and CO₂, and is used directly as feed to reactor system 301 in a preferred embodiment of the invention. As seen in the results of Table 4, the addition of CO₂ gave completely different unexpected results than the similar experiments of Examples 5 and 6 in which methanol was not present in the feed. The data from experiments 13-15, in which the DME/MeOH molar ratio in the reactor feed was about 10, indicate that CO₂ addition significantly increases the overall acetyl yield, but unexpectedly increases the selectivity of EDDA by a factor of three, while significantly decreasing the selectivity of acetic anhydride with little effect on the selectivity of methyl acetate.

The experiments were repeated with lower values of the DME/MeOH molar ratio in the range of 2.5-4.5, and the results for experiments 16-18 of Table 4 indicate an even greater increase in the EDDA selectivity as CO₂ is added, while acetyl yield increased at the higher value of CO₂ addition. Additional experiments were carried out at still lower values of the DME/MeOH ratio near 1.0. The results for experiments 19-20 given in Table 4 show that addition of CO₂ decreases the acetyl yield as well as the EDDA selectivity.

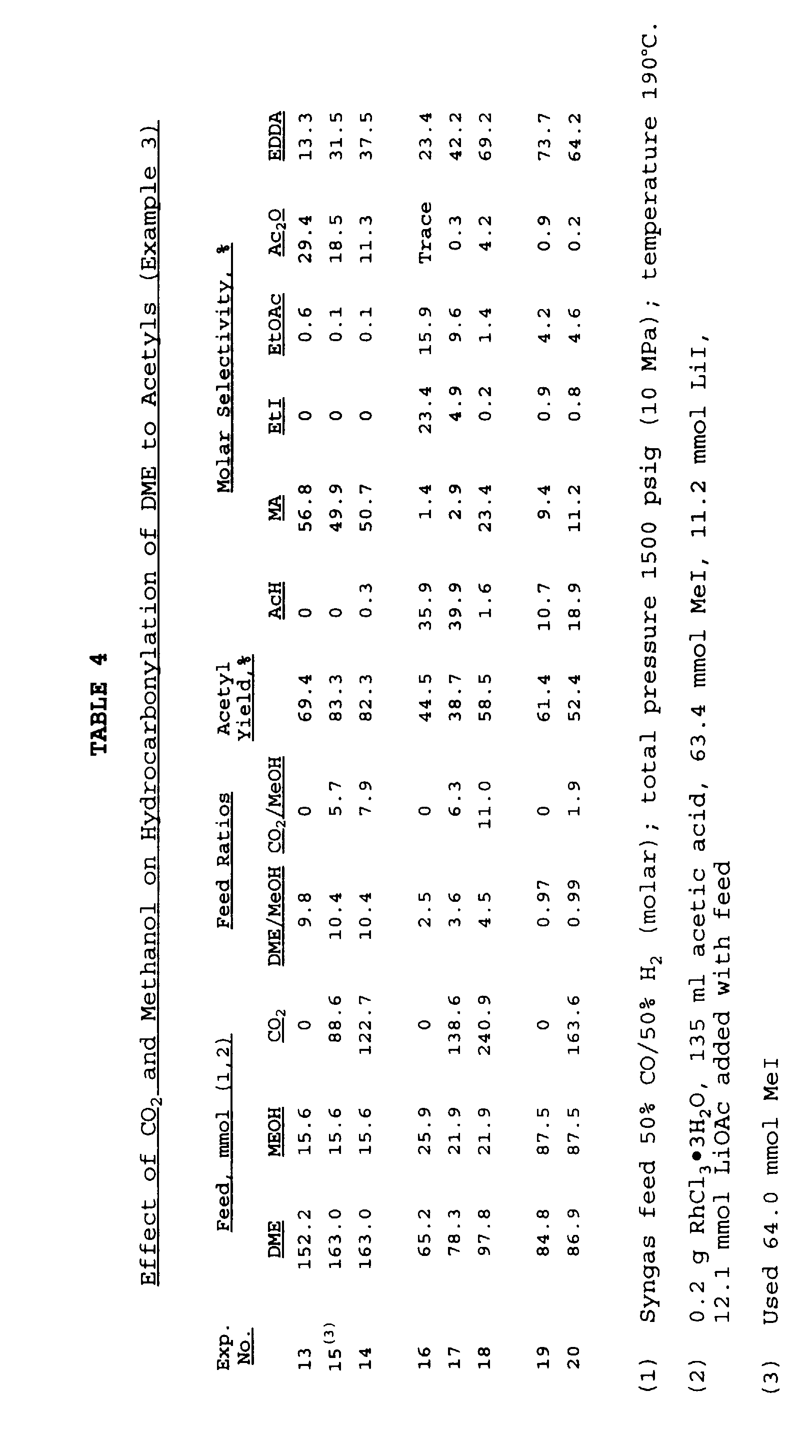

Thus when acetyl reactor feed is provided directly from the LP DME reactor and EDDA is a desired product, the inclusion of CO₂ in the reactor feed is a preferred mode of operation. The preferred range of the DME/MeOH molar ratio for improved EDDA selectivity is 3 to 11, although higher ratios are expected to give still higher EDDA selectivity. The range of the molar ratio CO₂/MeOH is 5 to 12, and more preferably 8 to 12. These preferred ranges can be realized when the feed to LP oxygenated acetyl reactor system 301 of Fig. 1 is provided directly from liquid phase dimethyl ether reactor system 201. This preferred mode of operation requires no additional treatment of effluent stream 19 of reactor system 201 as long as the water content is below 2 mol%. Water optionally can be removed by condensation if present at higher concentrations. Feed composition, catalyst composition, and operating conditions in DME reactor system 103 can be controlled to yield the preferred composition range in feed 25 to reactor system 301. Optionally CO₂ 28 can be added if required.

The present invention thus allows the production of oxygenated acetyl compounds directly from synthesis gas via the liquid phase dimethyl ether process followed directly by a liquid phase acetyl reactor system. Several variations to the disclosed process are possible within the scope of the present invention. These variations impart flexibility and utility to the process, and can be used in alternative applications depending on the desired mix of products and available feedstocks. The product slate can include one or more of the oxygenated acetyl compounds vinyl acetate, acetaldehyde, ethylidene diacetate, acetic acid, methyl acetate, and acetic anhydride. The operation of the acetyl reactor system with a feed containing methanol, DME, and syngas is improved unexpectedly by the inclusion of CO₂ in the feed, particularly when EDDA is the preferred reactor product. Unwanted coproducts are conveniently recycled to the POX reactor which reduces the POX feed requirements.

## Claims

1. A process for synthesizing one or more oxygenated acetyl compounds selected from ethylidene diacetate, acetaldehyde, acetic acid, acetic anhydride, and methyl acetate from a feed containing dimethyl ether, hydrogen, carbon monoxide, carbon dioxide and optionally methanol in a liquid phase reactor containing at least acetic acid in the presence of a catalyst system comprising a Group VIII metal, methyl iodide, lithium iodide, and optionally lithium acetate, characterized in that (a) lithium acetate is not present in the catalyst system and the molar ratio of carbon dioxide to dimethyl ether in said feed is between 0.3 and 1.3 or (b) lithium acetate is present in said catalyst, the molar ratio of carbon dioxide to methanol in said feed is between 5 and 12, and the molar ratio of dimethyl ether to methanol in said feed is between 3 and 11.

2. The use of carbon dioxide to improve the yield or selectivity of oxygenated acetyl compounds selected from ethylidene diacetate, acetaldehyde, acetic acid, acetic anhydride, and methyl acetate in an oxygenated acetyl process in which a feed containing dimethyl ether, hydrogen, carbon monoxide, and optionally methanol is reacted with acetic acid in a liquid phase reactor in the presence of a catalyst system comprising a Group VIII metal, methyl iodide, lithium iodide, and optionally lithium acetate.

3. A process of Claim 1 for the synthesis of ethylidene diacetate which comprises reacting a feed containing dimethyl ether, methanol, and synthesis gas which contains hydrogen, carbon monoxide, and carbon dioxide in a liquid phase oxygenated acetyl reactor containing at least acetic acid and a catalyst system comprising a Group VIII metal, methyl iodide, lithium iodide, and lithium acetate to react the components in said feed with acetic acid to produce said ethylidene diacetate and one or more additional oxygenated acetyl compounds, wherein the molar ratio of carbon dioxide to methanol in said feed is between 5 and 12, and the molar ratio of dimethyl ether to methanol in said feed is between 3 and 11.

4. A use of carbon dioxide of Claim 2 to improve the selectivity to ethylidene diacetate in an oxygenated acetyl process in which a feed containing dimethyl ether, methanol, hydrogen, and carbon monoxide is reacted with acetic acid in a liquid phase reactor in the presence of a catalyst comprising a Group VIII metal, methyl iodide, lithium iodide and lithium acetate.

5. A process of Claim 1 for synthesizing one or more oxygenated acetyl compounds selected from ethylidene diacetate, acetaldehyde, acetic acid, acetic anhydride, and methyl acetate from a feed containing dimethyl ether, hydrogen, carbon monoxide, carbon dioxide and optionally methanol in a liquid phase reactor containing at least acetic acid and a catalyst system comprising a Group VIII metal, methyl iodide, and lithium iodide but not lithium acetate, wherein carbon dioxide is present in said feed such that the molar ratio of carbon dioxide to dimethyl ether is between 0.3 and 1.3.

6. A use of carbon dioxide of Claim 2 to improve the yield of oxygenated acetyl compounds selected from ethylidene diacetate, acetaldehyde, acetic acid, acetic anhydride, and methyl acetate in an oxygenated acetyl process in which a feed containing dimethyl ether, hydrogen, carbon monoxide, and optionally methanol is reacted with acetic acid in a liquid phase reactor in the presence of a catalyst system comprising a Group VIII metal, methyl iodide, and lithium iodide but not lithium acetate.

7. A process or use of any one of Claims 1, 2 and 4 to 6, wherein said feed contains methanol and the molar ratio of dimethyl ether to methanol in said feed is between 3 and 11.

8. A process or use of any one of the preceding claims, wherein the molar ratio of carbon monoxide to hydrogen in said feed is between 0.6 and 4.0.

9. A process or use of any one of the preceding claims, wherein said Group VIII metal is rhodium (III) chloride trihydrate.

10. A process or use of any one of the preceding claims, wherein the liquid in said liquid phase reactor comprises one or more of said additional oxygenated acetyl compounds.

11. A process or use of any one of the preceding claims, wherein said feed to the liquid phase oxygenated acetyl reactor comprises a product stream obtained by reacting synthesis gas comprising hydrogen, carbon monoxide, and carbon dioxide in a liquid phase dimethyl ether reactor in the presence of a methanol synthesis catalyst and a methanol dehydration catalyst suspended in an inert liquid, said product stream comprising dimethyl ether, methanol, and unreacted synthesis gas.

12. A process or use of Claim 11, wherein additional carbon dioxide is added to said synthesis gas product stream.

## Patentansprüche

1. Verfahren zur Synthese einer oder mehrerer oxygenierter, aus Ethylidendiacetat, Acetaldehyd, Essigsäure, Essigsäureanhydrid und Methylacetat ausgewählter Acetylverbindungen aus einer Beschikkung, die Dimethylether, Wasserstoff, Kohlenmonoxid, Kohlendioxid und gegebenenfalls Methanol enthält, in einem Flüssigphasenreaktor, der mindestens Essigsäure enthält, in Gegenwart eines Katalysatorsystems, das ein Metall der Gruppe VIII, Methyliodid, Lithiumiodid und gegebenenfalls Lithiumacetat umfaßt, dadurch gekennzeichnet, daß (a) Lithiumacetat im Katalysatorsystem nicht vorhanden ist und das Molverhältnis von Kohlendioxid zu Dimethylether in der Beschickung zwischen 0,3 und 1,3 liegt oder (b) Lithiumacetat im Katalysator vorhanden ist, das Molverhältnis von Kohlendioxid zu Methanol in der Beschickung zwischen 5 und 12 liegt und das Molverhältnis von Dimethylether zu Methanol in der Beschickung zwischen 3 und 11 liegt.

2. Verwendung von Kohlendioxid zur Steigerung der Ausbeute oder Selektivität oxygenierter, aus Ethylidendiacetat, Acetaldehyd, Essigsäure, Essigsäureanhydrid und Methylacetat ausgewählter Acetylverbindungen in einem Verfahren für oxygeniertes Acetyl, bei dem eine Beschickung, die Dimethylether, Wasserstoff, Kohlenmonoxid und gegebenenfalls Methanol enthält, in einem Flüssigphasenreaktor in Gegenwart eines ein Metall der Gruppe VIII, Methyliodid, Lithiumiodid sowie gegebenenfalls Lithiumacetat enthaltenden Katalysatorsystems mit Essigsäure zur Umsetzung gebracht wird.

3. Verfahren nach Anspruch 1 zur Synthese von Ethylidendiacetat, bei dem eine Beschickung, die Dimethylether, Methanol und ein Wasserstoff, Kohlenmonoxid und Kohlendioxid enthaltendes Synthesegas enthält, in einem Flüssigphasenreaktor für oxygeniertes Acetyl, der mindestens Essigsäure und ein ein Metall der Gruppe VIII, Methyliodid, Lithiumiodid und Lithiumacetat enthaltendes Katalysatorsystem enthält, zur Umsetzung gebracht wird, um die Komponenten in der Beschickung mit Essigsäure umzusetzen, um das Ethylidendiacetat und eine oder mehrere zusätzliche oxygenierte Acetylverbindungen herzustellen, wobei das Molverhältnis von Kohlendioxid zu Methanol in der Beschickung zwischen 5 und 12 liegt und das Molverhältnis von Dimethylether zu Methanol in der Beschickung zwischen 3 und 11 liegt.

4. Verwendung von Kohlendioxid nach Anspruch 2, um die Selektivität für Ethylidendiacetat in einem Verfahren für oxygeniertes Acetyl zu verbessern, bei der eine Dimethylether, Methanol, Wasserstoff und Kohlenmonoxid enthaltende Beschickung in einem Flüssigphasenreaktor in Gegenwart eines ein Metall der Gruppe VIII, Methyliodid, Lithiumiodid und Lithiumacetat umfassenden Katalysators mit Essigsäure zum Umsetzung gebracht wird.

5. Verfahren nach Anspruch 1 zur Synthese einer oder mehrerer oxygenierter, aus Ethylidendiacetat, Acetaldehyd, Essigsäure, Essigsäureanhydrid und Methylacetat ausgewählter Acetylverbindungen aus einer Beschickung, die Dimethylether, Wasserstoff, Kohlenmonoxid, Kohlendioxid und gegebenenfalls Methanol enthält, in einem Flüssigphasenreaktor, der mindestens Essigsäure und ein ein Metall der Gruppe VIII, Methyliodid und Lithiumoxid, aber kein Lithiumacetat enthaltendes Katalysatorsystem umfaßt, wobei Kohlendioxid in einer solchen Menge in der Beschickung vorhanden ist, daß das Molverhältnis von Kohlendioxid zu Dimethylether zwischen 0,3 und 1,3 liegt.

6. Verwendung von Kohlendioxid nach Anspruch 2 zur Steigerung der Ausbeute oder Selektivität oxygenierter, aus Ethylidendiacetat, Acetaldehyd, Essigsäure, Essigsäureanhydrid und Methylacetat ausgewählter Acetylverbindungen in einem Verfahren für oxygeniertes Acetyl, bei dem eine Beschickung, die Dimethylether, Wasserstoff, Kohlenmonoxid und gegebenenfalls Methanol enthält, in einem Flüssigphasenreaktor in Gegenwart eines ein Metall der Gruppe VIII, Methyliodid und Lithiumiodid, aber kein Lithiumacetat enthaltenden Katalysatorsystems mit Essigsäure zur Umsetzung gebracht wird.

7. Verfahren oder Verwendung nach einem der Ansprüche 1, 2 und 4 bis 6, bei dem die Beschickung Methanol enthält und das Molverhältnis von Dimethylether zu Methanol in der Beschickung zwischen 3 und 11 liegt.

8. Verfahren oder Verwendung nach einem der vorstehenden Ansprüche, bei dem das Molverhältnis von Kohlenmonoxid zu Wasserstoff in der Beschickung zwischen 0,6 und 4,0 liegt.

9. Verfahren oder Verwendung nach einem der vorstehenden Ansprüche, bei dem das Metall der Gruppe VIII Rhodium(III)-chloridtrihydrat ist.

10. Verfahren oder Verwendung nach einem der vorstehenden Ansprüche, bei dem die Flüssigkeit im Flüssigphasenreaktor eine oder mehrere zusätzliche oxygenierte Acetylverbindungen umfaßt.

11. Verfahren oder Verwendung nach einem der vorstehenden Ansprüche, bei dem die Beschickung zum Flüssigphasenreaktor für oxygeniertes Acetyl einen Produktstrom umfaßt, der durch Umsetzung von Wasserstoff, Kohlenmonoxid und Kohlendioxid enthaltendem Synthesegas in einem Flüssigphasenreaktor für Dimethylether in Gegenwart eines Methanolsynthesekatalysators und eines Methanoldehydratisierungskatalysators, die in einer inerten Flüssigkeit suspendiert sind, erhalten wird, wobei der Produktstrom Dimethylether, Methanol und nicht umgesetztes Synthesegas umfaßt.

12. Verfahren oder Verwendung nach Anspruch 11, bei dem dem Synthesegasproduktstrom zusätzliches Kohlendioxid zugesetzt wird.

## Revendications

1. Procédé pour synthétiser un ou plusieurs composés acétyle oxygénés choisis parmi le diacétate d'éthylidène, l'acétaldéhyde, l'acide acétique, l'anhydride acétique et l'acétate de méthyle, à partir d'une alimentation contenant de l'éther diméthylique, de l'hydrogène, de l'oxyde de carbone, du gaz carbonique et éventuellement du méthanol, dans un réacteur opérant en phase liquide et contenant au moins de l'acide acétique, en présence d'un système catalyseur comprenant un métal du groupe VIII, de l'iodure de méthyle, de l'iodure de lithium et éventuellement de l'acétate de lithium, caractérisé (a) en ce que l'acétate de lithium n'est pas présent dans le système catalyseur et que le rapport molaire du gaz carbonique sur l'éther diméthylique dans ladite alimentation est entre 0,3 et 1,3 ou (b) en ce que de l'acétate de lithium est présent dans ledit catalyseur, que le rapport molaire du gaz carbonique sur le méthanol dans ladite alimentation est entre 5 et 12 et que le rapport molaire de l'éther diméthylique sur le méthanol dans ladite alimentation est entre 3 et 11.

2. Utilisation de gaz carbonique pour améliorer le rendement ou la sélectivité de la réaction de formation de composés acétyle oxygénés, choisis parmi le diacétate d'éthylidène, l'acétaldéhyde, l'acide acétique, l'anhydride acétique et l'acétate de méthyle, dans un procédé de production de composés acétyle oxygénés, dans lequel on fait réagir une alimentation contenant de l'éther diméthylique, de l'hydrogène, de l'oxyde de carbone et éventuellement du méthanol, avec de l'acide acétique, dans un réacteur opérant en phase liquide en présence d'un système catalyseur comprenant un métal du groupe VIII, de l'iodure de méthyle, de l'iodure de lithium et éventuellement de l'acétate de lithium.

3. Procédé selon la revendication 1, de synthèse de l'acétate d'éthylidène, qui comprend de faire réagir une alimentation contenant de l'éther diméthylique, du méthanol et un gaz de synthèse qui contient de l'hydrogène, de l'oxyde de carbone et du gaz carbonique, dans un réacteur opérant en phase liquide pour produire des composés acétyle oxygénés contenant au moins de l'acide acétique et un système catalyseur comprenant un métal du groupe VIII, de l'iodure de méthyle, de l'iodure de lithium et de l'acétate de lithium, pour faire réagir les composants de ladite alimentation avec de l'acide acétique, afin de produire ledit diacétate d'éthylidène et un ou plusieurs autres composés acétyle oxygénés additionnels, dans lequel le rapport molaire du gaz carbonique sur le méthanol dans ladite alimentation est entre 5 et 12 et dans lequel le rapport molaire de l'éther diméthylique sur le méthanol dans ladite alimentation est entre 3 et 11.

4. Utilisation de gaz carbonique selon la revendication 2 pour améliorer la sélectivité de la réaction de formation de diacétate d'éthylidène dans un procédé de production de composés acétyle oxygénés, dans lequel on fait réagir une alimentation contenant de l'éther diméthylique, du méthanol, de l'hydrogène et de l'oxyde de carbone avec de l'acide acétique dans un réacteur opérant en phase liquide, en présence d'un catalyseur comprenant un métal du groupe VIII, de l'iodure de méthyle, de l'iodure de lithium et de l'acétate de lithium.

5. Procédé selon la revendication 1, de synthèse d'un ou de plusieurs composés acétyle oxygénés choisis parmi le diacétate d'éthylidène, l'acétaldéhyde, l'acide acétique, l'anhydride acétique et l'acétate de méthyle, à partir d'une alimentation contenant de l'éther diméthylique, de l'hydrogène, de l'oxyde de carbone, du gaz carbonique et, le cas échéant du méthanol, dans un réacteur opérant en phase liquide contenant au moins de l'acide acétique et un système de catalyseur comprenant un métal du groupe VIII, de l'iodure de méthyle et de l'iodure de lithium, mais pas d'acétate de lithium, dans lequel le gaz carbonique est présent dans ladite alimentation en une quantité telle que le rapport molaire du gaz carbonique sur l'éther diméthylique se situe entre 0,3 et 1,3.

6. Utilisation de gaz carbonique selon la revendication 2, pour améliorer le rendement en composés acétyle oxygénés, choisis parmi le diacétate d'éthylidène, l'acétaldéhyde, l'acide acétique, l'anhydride acétique et l'acétate de méthyle, dans un procédé de production de composés acétyle oxygénés, dans lequel on fait réagir une alimentation contenant de l'éther diméthylique, de l'hydrogène, de l'oxyde de carbone et, le cas échéant, du méthanol, avec de l'acide acétique, dans un réacteur opérant en phase liquide, en présence d'un système catalyseur comprenant un métal du groupe VIII, de l'iodure de méthyle et de l'iodure de lithium, mais pas d'acétate de lithium.

7. Procédé ou utilisation selon l'une quelconque des revendications 1, 2 et 4 à 6, dans lequel ladite alimentation contient du méthanol et le rapport molaire de l'éther diméthylique sur le méthanol dans ladite alimentation est entre 3 et 11.

8. Procédé ou utilisation selon l'une quelconque des revendications précédentes, dans lequel ou dans laquelle le rapport molaire de l'oxyde de carbone sur l'hydrogène dans ladite alimentation est entre 0,6 et 4,0.

9. Procédé ou utilisation selon l'une quelconque des revendications précédentes, dans lequel ou dans laquelle ledit métal du groupe VIII est le chlorure de rhodium trihydrate.

10. Procédé ou utilisation selon l'une quelconque des revendications précédentes, dans lequel ou dans laquelle le liquide dans ledit réacteur opérant en phase liquide comprend un composé acétyle oxygéné additionnel ou davantage.

11. Procédé ou utilisation selon l'une quelconque des revendications précédentes, dans lequel ou dans laquelle ladite alimentation du réacteur opérant en phase liquide pour produire des composés acétyle oxygénés comprend un produit obtenu en faisant réagir un gaz de synthèse contenant de l'hydrogène, de l'oxyde de carbone et du gaz carbonique dans un réacteur opérant en phase liquide pour produire de l'éther diméthylique, en présence d'un catalyseur de synthèse du méthanol et d'un catalyseur de déshydratation du méthanol, en suspension dans un liquide inerte, ledit produit contenant de l'éther diméthylique, du méthanol et du gaz de synthèse n'ayant pas réagi.

12. Procédé ou utilisation selon la revendication 11, dans lequel ou dans laquelle du gaz carbonique additionnel est ajouté audit produit de réaction du gaz de synthèse.
